(19) Europäisches Patentamt
European Patent Office
Office européen des brevets

(11) **EP 1 734 073 B1**

(12) **EUROPÄISCHE PATENTSCHRIFT**

(45) Veröffentlichungstag und Bekanntmachung des Hinweises auf die Patenterteilung:
**15.05.2013 Patentblatt 2013/20**

(21) Anmeldenummer: **06011804.9**

(22) Anmeldetag: **08.06.2006**

(51) Int Cl.:
*C08L 5/00* *(2006.01)*     *C08J 3/03* *(2006.01)*
*C08J 3/05* *(2006.01)*     *C08L 1/00* *(2006.01)*
*C08L 3/00* *(2006.01)*

(54) **Verfahren zur Herstellung von sterilen Dextranaldehydlösungen**

Process for preparing sterile dextranaldehyde solutions

Procédé de préparation de solutions stériles de dextrane aldéhyde

(84) Benannte Vertragsstaaten:
**DE ES FR GB IT**

(30) Priorität: **17.06.2005 DE 102005030011**

(43) Veröffentlichungstag der Anmeldung:
**20.12.2006 Patentblatt 2006/51**

(73) Patentinhaber: **Aesculap AG**
**78532 Tuttlingen (DE)**

(72) Erfinder:
• **Odermatt, Erich K.**
**CH-8200 Schaffhausen (CH)**
• **Wegmann, Jürgen**
**78333 Stockach (DE)**

(74) Vertreter: **Patentanwälte**
**Ruff, Wilhelm, Beier, Dauster & Partner**
**Kronenstrasse 30**
**70174 Stuttgart (DE)**

(56) Entgegenhaltungen:
EP-A- 1 579 876     WO-A- 98/05366
WO-A- 03/035122     WO-A- 2006/042161
WO-A- 2006/050951     FR-A1- 2 754 268
US-A- 6 142 977     US-A1- 2003 032 622

EP 1 734 073 B1

**Beschreibung**

Die vorliegende Erfindung betrifft sterile Dextranaldehydlösungen sowie deren Herstellung.

Die Herstellung von pharmazeutischen und medizinischen Produkten auf der Basis von sterilisierten Polysaccharidlösungen ist von überragender Bedeutung, insbesondere für die Bereitstellung von flüssigen und gelartigen Hämostyptika, Gelen zur Adhäsionsprophylaxe sowie medizinischen Gewebeklebern.

Allgemein stehen für die Sterilisation von festen Polysacchariden und Polysaccharidmischungen eine Reihe von Methoden zur Verfügung, wobei die Entkeimung meistens durch Gamma- oder Elektronenbestrahlung oder durch Behandlung mit Ethylenoxid durchgeführt wird. Bezüglich der Sterilisation von wässrigen Polysaccharidlösungen weisen die herkömmlichen Sterilisationsmethoden jedoch einige Nachteile auf. So weisen die sterilisierten Produkte häufig die erwünschten Eigenschaften, beispielsweise Gelierung, entweder gar nicht oder lediglich in einem unbefriedigendem Ausmaß auf. Dies ist im Falle einer Sterilisation durch energiereiche Bestrahlung, beispielsweise Gamma- oder Elektronenbestrahlung, insbesondere darauf zurückzuführen, dass die Wassermoleküle in chemisch hochreaktive Hydroxyradikale gespalten werden, die wiederum weitere Radikalreaktionen in den gelösten Polysaccharidketten verursachen können. Auf diese Weise können Kettenabbrüche entstehen, die wiederum zu einer Reduzierung des Molekulargewichtes oder zu Vernetzungsreaktionen führen. Es handelt sich um nicht steuerbare Vorgänge, die die Eigenschaften der Polysaccharide sowie die Funktion der daraus hergestellten Produkte in unerwünschter Weise beeinträchtigen können.

Die Ethylenoxidsterilisation von wässrigen Polysaccharidlösungen besitzt den Nachteil, dass das toxische Ethylenoxid in Wasser löslich ist und daher dessen Entfernung nach dem Sterilisationsprozess problematisch ist. Entsprechendes gilt auch für chemische Sterilisationsverfahren, insbesondere mit Glutardialdehyd oder Formaldehyd, welche außerdem sehr reaktive Substanzen darstellen, die mit Polysacchariden unerwünschte Nebenreaktionen eingehen können.

Die Sterilfiltration stellt zwar eine leistungsstarke Sterilisationsalternative dar, allerdings nur für verdünnte wässrige Polysaccharidlösungen. Hochviskose Lösungen lassen sich dagegen, wenn überhaupt, nur mit einem geringen Durchfluss filtrieren. Die Filtermaterialien verblocken sehr schnell und müssen daher häufig gewechselt werden, so dass dieses Verfahren sehr hohe Kosten verursachen kann.

Zusätzlich zu den soeben erwähnten Nachteilen bei der Herstellung von sterilen wässrigen Polysaccharidlösungen stellt sich häufig das Problem der Auflösung der entsprechenden Polysaccharide in Wasser vor oder während des Sterilisationsprozesses, insbesondere bei schwerlöslichen Polysacchariden zur Herstellung von hoch konzentrierten wässrigen sterilen Polysaccharidlösungen. Zu deren Herstellung werden die Polysaccharide in Wasser üblicherweise bei höheren Temperaturen, insbesondere zwischen 60°C und 80°C, zum Teil über mehrere Tage gelöst. Die Lösungen werden anschließend langsam auf Raumtemperatur abgekühlt. Auf diese Weise werden übersättigte wässrige Lösungen der betreffenden Polysaccharide erhalten. Allerdings eignet sich dieses Verfahren lediglich für thermostabile Polysaccharide. Thermolabile Polysaccharide können dagegen infolge einer langen thermischen Behandlung insbesondere fragmentieren bzw. Seitenketten abspalten oder umlagern. Dies führt in den überwiegenden Fällen zu einem Verlust oder zumindest zu einer unerwünschten Beeinträchtigung der Eigenschaften der Polysaccharidlösungen bzw. der daraus hergestellten Produkte.

Aus der WO 2006/042161 A2 ist die Sterilisierung einer wäßrigen oxidierten Dextranlösung mit Hilfe von Gammabestrahlung bekannt. In der EP 1 579 876 A2 wird erwähnt, daß eine Zusammensetzung zur Adhäsionsprophylaxe, welche vorzugsweise eine wäßrige Lösung von Carboxymethylcellulose (CMC) aufweist, mittels Dampf sterilisiert werden kann. In der WO 2006/050951 A1 wird die Sterilisierung einer wäßrigen Dispersion von Hydroxymethylcellulose offenbart. Die WO 03/035122 A1 betrifft eine Zusammensetzung auf der Basis einer wäßrigen Lösung eines Aminogruppen-tragenden Polymers sowie einer wäßrigen Lösung eines Polyaldehyds zur Verklebung von biologischem Gewebe. Bei dem Polyaldehyd kann es sich insbesondere um Dextranaldehyd handeln. Aus der FR 2 754 268 geht eine Klebstoffzusammensetzung zur chirurgischen und/oder therapeutischen Verwendung hervor. Die Klebstoffzusammensetzung umfaßt einen bioabbaubaren, makromolekularen Polyaldehyd, bei welchem es sich bevorzugt um ein polyaldehydisches Polysaccharid handelt. Es wird beschrieben, daß eine wäßrige Lösung eines polyaldehydischen Polysaccharids mittels Sterilfiltration sterilisiert werden kann. In der WO 98/05366 A1 wird ein Verfahren zur terminalen Sterilisierung von befüllten Spritzen offenbart. Bei den zu sterilisierenden Spritzeninhalten handelt es sich bevorzugt um injizierbare Diagnostika, insbesondere Kontrastmittel.

Es stellt sich daher die Aufgabe, ein Verfahren zur Herstellung von wässrigen sterilen Polysaccharidlösungen aus thermolabilen und/oder schwerlöslichen Polysacchariden unter Umgehung der aufgeführten Nachteile des Standes der Technik bereitzustellen. Dabei sollen insbesondere die chemisch-physikalischen Eigenschaften der Polysaccharide bzw. Polysaccharidlösungen nicht beeinträchtigt werden, um ihre Anwendbarkeit in der Medizin und Chirurgie zu garantieren. Das Verfahren soll außerdem die wässrigen sterilen Polysaccharidlösungen in einer gebrauchsfertigen Form bereitstellen, deren Applikation insbesondere bei medizinischen und chirurgischen Eingriffen somit in einfacher Weise schnell und sicher erfolgen kann.

Diese Aufgabe wird gelöst durch ein Verfahren zur Herstellung von sterilen Dextranaldehydlösungen mit den Merkmalen von Anspruch 1 und durch eine Doppelkammerspritze mit den Merkmalen von Anspruch 12.

Im folgenden soll unter dem Begriff der Dispersion im Sinne der Erfindung ein System aus mindestens zwei Phasen verstanden werden, wobei eine Phase kontinuierlich und flüssig ist (Dispersionsmittel) und mindestens eine weitere Phase in Form eines fein verteilten Feststoffes vorliegt (dispergierte Phase). Daher umfasst der Begriff der Dispersion im Sinne der Erfindung ausdrücklich auch Suspensionen, Gele und Pasten.

Eine durch das erfindungsgemäße Verfahren hergestellte sterile Dextranaldehydlösung kann mit einer ebenfalls sterilen, insbesondere nach dem erfindungsgemäßen Verfahren sterilisierten, Chitosanlösung vermischt werden. Vorzugsweise werden eine Dextranaldehyd- und eine Chitosanlösung, insbesondere eine sterile 4%ige Chitosanlösung, vermischt. Das nach Mischen erhaltene Gel dient vorzugsweise als chirurgischer Kleber zum Gewebeverschluss und insbesondere zur Blutstillung.

Bevorzugt wird die wässrige unsterile Dispersion durch Mischen von Wasser und Dextranaldehyd hergestellt. Vorzugsweise wird die Dispersion durch Eintrag des Dextranaldehyds in Wasser hergestellt Die Herstellung der Dispersion wird in einer geeigneten Anlage, vorzugsweise einem Homogenisator, durchgeführt, welche mit einer bestimmten Menge Wasser beschickt wird, wobei das Wasser insbesondere aufbereitet, vorzugsweise voll entsalzt, ist. Mit Vorteil weist das Wasser wfi(water for injection)-Qualität auf. Auf diese Weise wird sichergestellt, dass das Sterilisationsgut nicht durch im Wasser gelöste Bestandteile, beispielsweise Salze und Metalle, in irgendeiner Weise beeinträchtigt wird.

Alternativ dazu kann die wässrige unsterile Dispersion auch aus einem Wasser/DMSO-Gemisch, insbesondere durch Eintrag des Dextranaldehyds in ein derartiges Gemisch, hergestellt werden. Vorzugsweise werden Wasser/DMSO-Gemisch in einem Verhältnis von 99,5 : 0,5 Vol-%. bis 50 : 50 % Vol-%, insbesondere von 99,5 : 0,5 Vol-% bis 90 : 10 Vol-%, verwendet. Bezüglich weiterer Merkmale der Wasser/DMSO-Gemische wird auf die obige Beschreibung verwiesen.

Erfindungsgemäß kann der Eintrag des Dextranaldehyds in Wasser oder in ein Wasser/DMSO-Gemisch während einer Zeit zwischen 60 min und 120 min, insbesondere zwischen 80 min und 100 min, vorzugsweise während ca. 90 min, vorgenommen werden. Vorteilhafterweise erfolgt die Herstellung der Dispersion, insbesondere der Eintrag des Dextranaldeyhds in Wasser, bei einer Temperatur zwischen 4°C und 25°C, insbesondere zwischen 4°C und 15°C, vorzugsweise bei ca. 6°C.

In einer weiteren Ausführungsform des erfindungsgemäßen Verfahrens wird zur Herstellung der wässrigen Dispersion lyophilisierter Dextranaldehyd verwendet.

Mit Vorteil wird die insbesondere nach den bisher beschriebenen Schritten des erfindungsgemäßen Verfahrens erhaltene wässrige Dispersion homogenisiert. Dies kann vorzugsweise nach beendeter Zugabe des Dextranaldehyds zu dem in der bereits erwähnten Anlage, insbesondere in dem Homogenisator, vorgelegten und vorzugsweise aufbereiteten, insbesondere entsalzten, Wasser innerhalb eines Zeitraumes von beispielsweise ca. 30 min erfolgen.

Die wässrige Dispersion wird bevorzugt mit einem Gehalt an Dextranaldehyd von 5 bis 20 Gew.-%, insbesondere 5 bis 15 Gew.-%, vorzugsweise von ca. 10 Gew.-%, hergestellt.

Das erfindungsgemäße Verfahren zeichnet sich vorzugsweise weiterhin dadurch aus, dass die wässrige Dispersion luftdicht und insbesondere luftfrei verpackt wird. Dies ist besonders vorteilhaft für den Sterilisationserfolg, da auf diese Weise reproduzierbare Temperatur/Druckverhältnisse erzeugt werden.

In einer besonders bevorzugten Ausführungsform wird die Dispersion der wässrigen Dextranaldehydlösung in mindestens ein geschlossenes Behältnis, vorzugsweise in eine Ein- oder Zweikammerspritze, überführt. Eine Zweikammer- bzw. Zwillingsspritze ist als geschlossenes Behältnis besonders vorteilhaft, da auf diese Weise gleichzeitig zwei verschiedene wässrige Polysacchariddispersionen getrennt sterilisiert und insbesondere bei einem chirurgischen Eingriff durch den bestimmungsgemäßen Gebrauch der Spritze als Mischung auf den gewünschten Bestimmungsort appliziert werden können. Bevorzugt liegt eine der getrennt zu sterilisierenden wässrigen Dispersionen in Form einer Lösung vor. Vorzugsweise enthält die eine Kammer der Zweikammerspritze die Dispersion von Dextranaldehyd und die andere Kammer der Zweikammerspritze die Lösung eines Aminogruppentragenden Polysaccharids, insbesondere Chitosan. Besonders bevorzugt enthält die eine Kammer eine 4%ige Chitosanlösung. Die nach der thermischen Behandlung vorliegenden sterilen Polysaccharidlösungen, insbesondere eine sterile Dextranaldehyd- und eine sterile Chitosanlösung, beispielsweise eine 4%ige Chitosanlösung, können, wie bereits erwähnt, zu einem chirurgischen Gewebekleber mit insbesondere blutstillender Wirkung vermischt werden.

In einer besonders bevorzugten Ausführungsform des erfindungsgemä-βen Verfahrens wird die insbesondere nach den bisher erwähnten Schritten hergestellte wässrige Dispersion in Form einer Paste oder eines Gels sterilisiert, wobei die Dispersion zunächst als Suspension vorliegen kann, die sich vor dem Sterilisieren bei längerem Stehenlassen, beispielsweise innerhalb von 2 bis 3 Stunden, in eine Paste oder ein Gel umwandelt. Erfindungsgemäß kann es aber ebenso vorgesehen sein, dass die Suspension unmittelbar einer thermischen Behandlung und damit einer Sterilisation unterworfen wird.

In besonders vorteilhafter Weise wird bei dem vorliegenden erfindungsgemäßen Verfahren die Auflösung des Dextranaldehyds und die Sterilisation gleichzeitig durch Behandlung der verpackten wässrigen Dispersion mit einem verdampfbaren Medium bewirkt, das unter erhöhtem Druck mühelos insbesondere die standardisierten Sterilisationstemperaturen, insbesondere von 121°C bzw. 134°C, erreicht. Einerseits wirkt das verdampfte Medium als Wärmeüberträger auf das Wasser der verpackten Dispersion und andererseits als Druckausgleich. Durch die Kondensation des Wasserdampfes an dem kühleren verpackten Sterilisationsgut, insbesondere dem Dextranaldehyd, wird die Kondensationsenergie des Wassers an das Sterilisationsgut schnell abgegeben. Dies führt zum Aufheizen des Sterilisationsgutes und zum Absterben von gegebenenfalls in der wässrigen Dispersion bzw. Lösung anwesenden Mikroorganismen. Beim bevorzugten Arbeiten mit gesättigtem Dampf wirkt das Medium als Heizmedium und Druckbildner, der als Gegendruck den in der verpackten wässrigen Dispersion durch die Temperaturerhöhung erzeugten Druck ausgleicht.

In einer besonders bevorzugten Ausführungsform des erfindungsgemä-βen Verfahrens handelt es sich bei dem verdampfbaren Medium um Wasser, so dass die Auflösung und die Sterilisation des Dextranaldehyds durch Behandlung der verpackten wässrigen Dispersion mit Wasserdampf bewirkt wird.

Das erfindungsgemäße Verfahren kennzeichnet sich in vorteilhafter Weise dadurch aus, dass die thermische Behandlung mit dem verdampften Medium, vorzugsweise mit Wasserdampf, im wesentlichen in Abwesenheit von Luft und damit in einer reinen Dampfatmosphäre des Mediums, vorzugsweise in einer reinen Wasserdampfatmosphäre, durchgeführt wird.

Die thermische Behandlung wird während einer Dauer von 5 min bis 30 min vorgenommen. Vorzugsweise erfolgt die thermische Behandlung während einer Dauer von 20 min.

Die thermische Behandlung wird zwischen mehr als 100°C und 140°C, insbesondere zwischen 115°C und 125°C, vorzugsweise bei 121°C, durchgeführt.

Die thermische Behandlung wird bei einem Überdruck zwischen 1 bar und 5 bar, insbesondere zwischen 1 bar und 3 bar, vorzugsweise bei 1 bar oder 2 bar, durchgeführt.

Mit Vorteil werden für die Sterilisation der wässrigen Dextranaldehyddispersion standardisierte Sterilisationsbedingungen angewendet. Vorzugsweise erfolgt die Sterilisation bei einer Temperatur von 121°C und einem Druck von 1 bar während 20 min. Es kann aber auch bevorzugt sein, die Sterilisation der Dextranaldehyddispersion bei einer höheren Temperatur während einer kürzeren Behandlungszeit durchzuführen. Dazu ist ein höherer Druck erforderlich. Ein derartig ausgerichtetes standardisiertes Sterilisationsprotokoll sieht eine Temperatur von 134°C während 5 min bei einem Druck von 2 bar vor. Während eine Sterilisationstemperatur von 121°C für das Material der meisten Verpackungen, insbesondere von Spritzen, unproblematisch ist, können höhere Sterilisationstemperaturen, insbesondere eine Sterilisationstemperatur von 134°C, zu erheblichen Problemen bei herkömmlichen Verpackungsmaterialien führen. Derartige Probleme können durch die Verwendung geeigneter Materialien, insbesondere Kunststoffe, beseitigt werden. So können als Materialien, insbesondere für Spritzen, Cycloolefin-Copolymere, vorzugsweise aus Norbornen und Ethylen, verwendet werden, die insbesondere unter der Bezeichnung Topas® (Thermoplastic Olefin Polymer of Amorphous Structure) kommerziell vertrieben werden.

Erfindungsgemäß kann es ferner vorgesehen sein, dass die thermische Behandlung, vorzugsweise mit Wasserdampf, in einem Autoklaven, insbesondere in einem Sterilisationsautoklaven, durchgeführt wird. Derartige Autoklaven weisen in vorteilhafter Weise bereits standardisierte Sterilisationsprogramme, insbesondere bei einer Temperatur von 121°C bzw. 134°C, auf.

Das erfindungsgemäße Verfahren zeichnet sich gegenüber herkömmlichen Sterilisationsverfahren dadurch aus, dass die gewünschten, Eigenschaften des zu sterilisierenden Dextranaldehyds, insbesondere die chemisch-physikalischen Eigenschaften, durch den Sterilisationsvorgang nicht bzw. nicht in negativer Weise beeinträchtigt werden. Im Vergleich zu herkömmlichen, insbesondere thermischen, Lösungsverfahren zeichnet sich das erfindungsgemäße Verfahren auch durch eine kürzere Auflösungszeit für Dextranaldehyd aus, wodurch sich insgesamt kürzere Herstellungszeiten für sterile Dextranaldehydlösungen ergeben. Gleichzeitig werden die sterilen Dextranaldehydlösungen durch das erfindungsgemäße Verfahren in einer gebrauchsfertigen Form, vorzugsweise für medizinische und chirurgische Applikationsfelder, bereitgestellt, die eine einfache und sichere Handhabung durch den Anwender, insbesondere Chirurgen, erlaubt. Ein aufwendiger und teurer aseptischer Abfüllungsvorgang, der sich bei vielen herkömmlichen Sterilisationsverfahren, insbesondere der Sterilfiltration, notwendigerweise anschließt und au-βerdem ein nicht unerhebliches Verkeimungsrisiko bedeutet, wird auf diese Weise überflüssig.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

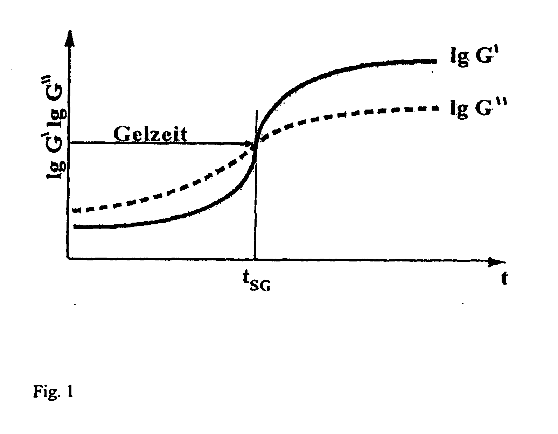

Fig. 1:    Schematische Darstellung des zeitlichen Verlaufs der viskosen Anteile (G") und der elastischen Anteile (G') während der Gelierungsreaktion zwischen Dextranaldehyd und Chitosan. Der Schnittpunkt der beiden Kurven entspricht der Gelzeit.

<u>Beispiele:</u>

<u>1. Herstellung einer wässrigen Polymerpaste</u>

In einer Process-Pilot Anlage der Fa. IKA Werke (Staufen, Deutschland) werden ein 2-Generator-Pumpstufen-System und ein Generator eingebaut. Insgesamt werden 2 L Pharmawasser (B/Braun, Melsungen Deutschland) vorgelegt. Vorlagegefäß und Homogenisator werden auf 6°C gekühlt. In einem Zeitraum vom 90 Minuten werden kontinuierlich 200 g lyophilisiertes Dextranaldehyd zugegeben. Nach beendeter Zugabe wird die resultierende wässrige Polymerpaste noch 30 Minuten homogenisiert und in 5 mL Ein- und Zweikammer-Spritzen abgefüllt.

<u>2. Dampfsterilisation einer wässrigen Polymerpaste</u>

Die Spritzen werden in TYVEK-Beutel verpackt in eine Spritzenhalterung eingespannt und im Autoklaven dampfsterilisiert. Hierzu wird ein Standardprogramm von 20 Minuten bei 121°C verwendet. Durch die Sterilisation wandelt sich das pastöse Polymergel in eine durchsichtige Flüssigkeit um.

<u>3. Nachweis der Sterilität</u>

In jeweils 20 mit Dextranaldehydpaste (DA-Paste) abgefüllte Ein- und Zweikammerspritzen wurden vor der Sterilisation Spore Strips gegeben, welche Sporen des Bakteriums Geobacillus stearothermophilus mit einem Gehalt von $1 \times 10^6$ Sporen enthielten. Die Spritzen wurden wie in Bsp. 2 beschrieben sterilisiert und anschließend auf Sterilität überprüft. Die Tests wurden nach den Anforderungen der Ph. Eur. 4 (2004) mittels Direktbeschickung durchgeführt. Hierzu wurden die Filter 14 Tage in Caso-Bouillon bei 20-25°C und in Thioglykolat-Bouillon bei 30-35°C inkubiert. Sämtliche Spritzen enthielten flüssige und sterile Dextranaldehydlösungen.

<u>4. Herstellung einer übersättigten Dextranaldehydlösung (DA-Lösung),(Vergleichsversuch)</u>

100 g Dextranaldehyd werden in 1000 ml Pharmawasser gegeben und bei 60°C gerührt. Nach ca. 96 Stunden hat sich das Dextranaldehyd vollständig aufgelöst. Die klare Lösung wurde langsam auf Raumtemperatur abgekühlt.

<u>5. Sterilisation der DA-Lösung (Vergleichsversuch)</u>

Die vollständig gelöste DA-Lösung aus Bsp. 4 wird in Ein- oder Zweikammerspritzen abgefüllt, in TYVEK-Beutel verpackt, in eine Spritzenhalterung eingespannt und im Autoklaven dampfsterilisiert. Hierzu wird ein Standardprogramm von 20 Minuten bei 121°C verwendet.

<u>6. Konzentration der Lösungen</u>

Der Wassergehalt der eingesetzten lyophilisierten Dextranaldehyd-Vliese wurde mittels Karl Fischer Titration in einer Fünffachbestimmung bestimmt. Für die Vliese konnte ein Wassergehalt von 20% ermittelt werden. Die in Beispiel 1 und 4 hergestellten Lösungen haben einen Dextranaldehydgehalt von 7,8% (w/v). In einer Dreifachbestimmung wurde durch gravimetrische Messungen der Wassergehalt der sterilen Lösungen (Bsp 2) und der Paste (Bsp.1) bestimmt.

|  | DA-Paste (Bsp. 1) | Sterile DA-Lösung (Bsp. 2) |
|---|---|---|
| Gehalt Dextranaldehyd [%] | 7,5 ± 0,6 % | 7,6 ± 0,3 % |

<u>7. Bestimmung des Aldehydgehalts</u>

Der Einfluss der Sterilisation auf die chemische Struktur wurde durch Bestimmung des Aldehydgehalts durchgeführt. Hierzu wurden die aus der Paste sterilisierten Lösungen erneut lyophilisiert. Die hergestellten Lyophilisate wurden titrimetrisch auf den Anteils an oxidierten Glucoseeinheiten überprüft [B.T. Hofreiter, B.H. Alexander, I.A. Wolff, Anal. Chem. 1955, 27, 1930ff.].

0,15 g Dextranaldehyd (DA) werden in einem Erlenmeyerkolben vorgelegt und anschließend mit 10 ml einer 0,25 N carbonatfreien NaOH Lösung versetzt. Die Mischung wird gerührt bis das eingesetzte Dextranaldehyd gelöst ist. Danach wird der Kolben für eine Minute in ein heißes Wasserbad (80°C) getaucht und anschließend unter starkem Rühren ins Eisbad gestellt. Nach einer Minute werden unter Rühren vorsichtig 15 ml 0,25 N Schwefelsäure hinzugegeben.

Die Mischung wird anschließend mit 50 ml Wasser verdünnt und mit 1 ml 0,2%iger Phenolphthaleinlösung versetzt. Die saure Lösung wird mit 0,25 N NaOH Lösung gegen den Indikator titriert.

Aus der zugegebenen Mengen an Dextran bzw. Dextranaldehyd sowie dem Verbrauch an Säure und Base berechnet sich der Anteil an oxidierten Glucoseeinheiten X wie folgt:

$$X = \left[ \frac{\left(n_{eqBase} - n_{eqSäure}\right)_{DA}}{\frac{W_{DA}}{161}} - \frac{\left(n_{eqBase} - n_{eqSäure}\right)_{Dextran}}{\frac{W_{Dextran}}{162}} \right] \times 100\%$$

X: Dialdehydgehalt
$n_{eqSäure}$: Äquivalenzstoffmenge der Säure
$n_{eqBase}$: Äquivalentstoffmenge der Base
$W_{DA}$: Trockengewicht Dextranaldehyd
$W_{Dextran}$: Trockengewicht Dextran
$n_{NaOH}$: Normalität des NaOH Titers
$n_{H2SO4}$: Normalität der verwendeten $H_2SO_4$-Lösung

|  | Vor Herstellung der Paste | Nach Sterilisation |
|---|---|---|
| Anteil an oxidierten Glucoseeinheiten [%] | 96,6 ± 1,4 | 94,7 ± 0,9 |

Fazit aus Bsp. 6 und 7: Durch das neue Verfahren werden chemische Struktur und Konzentration nicht verändert.

8. Vergleich der Gelzeiten mit Chitosanlösungen

Dextranaldehydlösung und Chitosan bilden beim Mischen ein Gel aus, welches als chirurgischer Gewebekleber u.a.zur Blutstillung verwendet werden kann. Hierbei ist eine schnelle Gelbildung (< 10 s) für den Chirurgen von entscheidender Bedeutung, damit dieses nicht aus dem Wundbereich gespült wird.

Zur Bestimmung der Gelzeit wurden 10 Doppelkammerspritzen mit 5 mL Gesamtvolumen (Mixpac Systems, Rotkreuz Schweiz) jeweils mit 1 ml einer wässrigen 4%igen Chitosanlösung (Protasan® Fa. FMC Biopolymers, Drammen Norwegen) und 1 ml Polymerpaste (Bsp. 1)gefüllt. Parallel hierzu wurden 10 weitere Doppelkammerspritzen mit 1 ml einer 4%igen Chitosanlösung und 1 ml Dextranaldehydlösung (Bsp. 4) gefüllt. Die Spritzen wurden, wie unter Bsp. 2 bzw. Bsp. 5 beschrieben, einer Dampfsterilisation unterzogen.

Die Gelzeit wurde mit einem Gemini 150 Rheometer untersucht (Malvern Instruments, Herrenberg Deutschland). Mit einem Platte-Platte Messsystem wurde der zeitliche Verlauf des Gelierungsprozesses in Oszillation bestimmt, indem auf die Doppelkammerspritze eine statische Mischspritze (Mixpac Systems, Rotkreuz Schweiz) aufgesetzt und die Mischung zwischen die Messplatten eingespritzt wurde.

|  | Unsterile DA-Lösung (Bsp. 4) (Stand der Technik) | Sterile DA-Lösung hergestellt durch Sterilisation der Paste (Bsp.2) | Sterile DA-Lösung (Bsp.5) (Stand der Technik) |
|---|---|---|---|
| Gelzeit mit steriler 4%iger Protasanlösung | 5,2 s ± 1,5 s | 7,0 s ± 1,3 s | 78,6 ± 26,7 s |

9. Kohäsive Kräfte der medizinischen Gele:

Die Bestimmung des Verlustfaktors δ wurde ebenfalls mit dem Gemini 150 Rheometer bestimmt.

$$\tan \delta = G''/G'$$

| | Unsterile DA-Lösung (Bsp. 4) (Stand der Technik) | Sterile DA-Lösung hergestellt durch Sterilisation der Paste (Bsp.2) | Sterile DA-Lösung (Bsp.5) (Stand der Technik) |
|---|---|---|---|
| Verlustfaktor $\delta$ 60 s nach Gelzeit | 13,2 $\pm$ 2,4 | 8,3 $\pm$ 1,1 | 28,0 $\pm$ 2,0 |
| Verlustfaktor $\delta$ 120 s nach Gelzeit | 6,9 $\pm$ 1,5 | 4,3 $\pm$ 1,6 | 14,1 $\pm$ 2,1 |

Fazit aus Bsp. 8 und 9:

Das neuartige Sterilisationsverfahren der DA-Paste führt zu geringeren Gelzeiten und deutlich höheren kohäsiven Kräften im Vergleich zur Sterilisation der DA-Lösung.

10. Zeit zwischen Herstellung der Paste und Durchführung der Sterilisation

Die milchig weiße Paste verändert bei längerer Standzeit ihre Konsistenz zu einem transparenten Gel. Der Einfluss der Standzeit auf die Eigenschaften der resultierenden sterilen Lösung wurde untersucht. Hierzu wurde eine 2 l Polymerpaste (Bsp 1) in 4 Fraktionen geteilt, welche nach 1, 4, 7 bzw. 9 Tagen nach Pastenherstellung sterilisiert wurden. Die entsprechenden Pasten wurden nach den Beispielen charakterisiert:

| Standzeit zwischen Pastenherstellung und Dampfsterilisation | Konzentration % (w/v) (Bsp. 6) | Anteil an oxidierten Glucoseeinheiten (Bsp.7) |
|---|---|---|
| 1 Tag | 8,1 $\pm$ 0,5% | 93,7 $\pm$ 1,0% |
| 4 Tage | 7,3 $\pm$ 0,3 % | 94,7 $\pm$ 0,9 % |
| 7 Tage | 7,8 $\pm$ 0,8 % | 93,2 $\pm$ 0,9 % |
| 9 Tage | 7,8 $\pm$ 0,2 % | 93,0 $\pm$ 0,5 % |

| Standzeit zwischen Pastenherstellung und Dampfsterilisation | Gelzeit mit 4%iger steriler Chitosanlösung (Bsp.8) | Phasenverschiebungswinkel $\delta$ nach 60 s (Bsp.9) |
|---|---|---|
| 1 Tag | 7,2 $\pm$ 0,8 s | 4,2 $\pm$ 0,4 |
| 4 Tage | 7,5 $\pm$ 1,6 s | 3,9 $\pm$ 0,8 |
| 7 Tage | 8,8 $\pm$ 0,9 s | 3,5 $\pm$ 0,4 |
| 9 Tage | 9,2 $\pm$ 1,9% | 3,0 $\pm$ 0,3 |

Fazit: Die Standzeit zwischen Pastenherstellung und Dampfsterilisation hat keinen Einfluss auf die Eigenschaften der resultierenden sterilen Dextranaldehydlösung.

11. Strahlensterilisation der Polymerpaste:

Die Polymerpaste aus Bsp 1 wurde alternativ zur Dampfsterilisation auch mit E-Beam und Gamma-Strahlung bestrahlt. Die verwendeten Dosen betrugen 18 und 25 kGy. Die Polymerpasten waren nach der Sterilisation gelöst. Die Gelierung mit 4%iger Chitosanlösung erfolgte jedoch deutlich langsamer. Gleichzeitig sind die kohäsiven Kräfte der resultierenden Lösungen deutlich geringer.

| Sterilisationsart der 10%igen Paste | Gelzeit mit 4%iger steriler Chitosanlösung (Bsp.8) | Phasenverschiebungswinkel $\delta$ nach 60 s (Bsp.9) |
|---|---|---|
| E-Beam 18 kGy | 13,4 $\pm$ 2,4 s | 20,1 $\pm$ 1,1 |
| E-Beam 25 kGy | 30,9 $\pm$ 4,7 s | 26,8 $\pm$ 1,9 |
| Gamma 18 kGy | 24,4 $\pm$ 4,2 s | 20,6 $\pm$ 1,3 |
| Gamma 25 kGy | 55,9 $\pm$ 15,3% | 35,3 $\pm$ 4,7 |

Fazit:

Die Dampfsterilisation ist der Strahlensterilisation vorzuziehen.

**Patentansprüche**

1. Verfahren zur Herstellung von sterilen Dextranaldehydlösungen, **dadurch gekennzeichnet, dass** mindestens eine wässrige unsterile Dextranaldehyddispersion verpackt wird und die Auflösung des Dextranaldehyds und die Sterilisation gleichzeitig durch thermische Behandlung der Verpackung bewirkt werden, wobei die thermische Behandlung zwischen mehr als 100°C und 140°C, während einer Dauer von 5 bis 30 min und bei einem Druck zwischen 1 und 5 bar vorgenommen wird.

2. Verfahren nach Anspruch 1, **dadurch gekennzeichnet, dass** die Dispersion durch Eintrag des Dextranaldehyds in Wasser oder in ein Wasser/DMSO-Gemisch hergestellt wird.

3. Verfahren nach Anspruch 2, **dadurch gekennzeichnet, dass** der Eintrag während einer Zeit zwischen 60 min und 120 min vorgenommen wird.

4. Verfahren nach einem der vorhergehenden Ansprüche, **dadurch gekennzeichnet, dass** die Herstellung der Dispersion bei einer Temperatur zwischen 4 °C und 25 °C durchgeführt wird.

5. Verfahren nach einem der vorhergehenden Ansprüche, **dadurch gekennzeichnet, dass** die Dispersion mit einem Gehalt an Dextranaldehyd von 5 bis 20 Gew.-% hergestellt wird.

6. Verfahren nach einem der vorhergehenden Ansprüche, **dadurch gekennzeichnet, dass** die Dispersion luftdicht verpackt wird.

7. Verfahren nach einem der vorhergehenden Ansprüche, **dadurch gekennzeichnet, dass** die mindestens eine Dispersion in ein geschlossenes Behältnis überführt wird.

8. Verfahren nach einem der vorhergehenden Ansprüche, **dadurch gekennzeichnet, dass** die Dispersion in Form einer Paste oder eines Gels sterilisiert wird.

9. Verfahren nach einem der vorhergehenden Ansprüche, **dadurch gekennzeichnet, dass** die Auflösung und Sterilisierung durch Behandlung der verpackten Dispersion mit Wasserdampf bewirkt wird.

10. Verfahren nach einem der vorhergehenden Ansprüche, **dadurch gekennzeichnet, dass** die thermische Behandlung mit Wasserdampf im wesentlichen in Abwesenheit von Luft durchgeführt wird.

11. Verfahren nach einem der vorhergehenden Ansprüche, **dadurch gekennzeichnet, dass** die thermische Behandlung mit Wasserdampf in einem Autoklaven durchgeführt wird.

12. Doppelkammerspritze enthaltend eine sterile Dextranaldehydlösung, hergestellt nach einem der vorhergehenden Ansprüche und eine sterile Chitosanlösung.

## Claims

1. Method for preparing sterile dextran aldehyde solutions, **characterized in that** at least one aqueous non-sterile dextran aldehyde dispersion is packed and the dissolution of the dextran aldehyde and the sterilization are brought about simultaneously by thermal treatment of the packing, wherein the thermal treatment is performed at between more than 100°C and 140°C over a period of from 5 to 30 min and at a pressure of between 1 and 5 bar.

2. Method according to Claim 1, **characterized in that** the dispersion is prepared by introduction of the dextran aldehyde into water or into a water/DMSO mixture.

3. Method according to Claim 2, **characterized in that** the introduction is performed over a time of between 60 min and 120 min.

4. Method according to any of the preceding claims, **characterized in that** the preparation of the dispersion is carried out at a temperature of between 4°C and 25°C.

5. Method according to any of the preceding claims, **characterized in that** the dispersion is prepared with a dextran aldehyde content of from 5 to 20% by weight.

6. Method according to any of the preceding claims, **characterized in that** the dispersion is packed in an airtight manner.

7. Method according to any of the preceding claims, **characterized in that** the at least one dispersion is transferred to a closed container.

8. Method according to any of the preceding claims, **characterized in that** the dispersion is sterilized in the form of a paste or a gel.

9. Method according to any of the preceding claims, **characterized in that** the dissolution and sterilization is brought about by treatment of the packed dispersion with water vapour.

10. Method according to any of the preceding claims, **characterized in that** the thermal treatment with water vapour is carried out substantially in the absence of air.

11. Method according to any of the preceding claims, **characterized in that** the thermal treatment with water vapour is carried out in an autoclave.

12. Double-chamber syringe containing a sterile dextran aldehyde solution prepared according to any of the preceding claims and a sterile chitosan solution.

## Revendications

1. Procédé pour la préparation de solutions stériles de dextrane-aldéhyde, **caractérisé en ce qu'**on place dans un emballage au moins une dispersion aqueuse de dextrane-aldéhyde non stérile et on provoque simultanément la dissolution du dextrane-aldéhyde et la stérilisation par traitement thermique de l'emballage, le traitement thermique étant effectué entre plus de 100 °C et 140 °C, pendant une durée de 5 à 30 min et sous une pression comprise entre 1 et 5 bars.

2. Procédé selon la revendication 1, **caractérisé en ce qu'**on prépare la dispersion par introduction du dextrane-aldéhyde dans de l'eau ou dans un mélange d'eau/DMSO.

3. Procédé selon la revendication 2, **caractérisé en ce qu'**on effectue l'introduction pendant une durée comprise entre 60 min et 120 min.

4. Procédé selon l'une quelconque des revendications précédentes, **caractérisé en ce qu'**on effectue la préparation de la dispersion à une température comprise entre 4 °C et 25 °C.

5. Procédé selon l'une quelconque des revendications précédentes, **caractérisé en ce qu'**on prépare la dispersion à une teneur en dextrane-aldéhyde de 5 à 20 % % en poids.

6. Procédé selon l'une quelconque des revendications précédentes, **caractérisé en ce qu'**on place la dispersion dans un emballage étanche à l'air.

7. Procédé selon l'une quelconque des revendications précédentes, **caractérisé en ce que** ladite au moins une dispersion est transférée dans un contenant fermé.

8. Procédé selon l'une quelconque des revendications précédentes, **caractérisé en ce que** la dispersion est stérilisée sous forme d'une pâte ou d'un gel.

9. Procédé selon l'une quelconque des revendications précédentes, **caractérisé en ce qu'**on provoque la dissolution et la stérilisation par traitement par de la vapeur d'eau de la dispersion emballée.

10. Procédé selon l'une quelconque des revendications précédentes, **caractérisé en ce que** le traitement thermique est effectué avec de la vapeur d'eau essentiellement en absence d'air.

11. Procédé selon l'une quelconque des revendications précédentes, **caractérisé en ce que** le traitement thermique est effectué avec de la vapeur d'eau dans un autoclave.

12. Seringue à double chambre, contenant une solution stérile de dextrane-aldéhyde, préparée selon l'une quelconque des revendications précédentes, et une solution stérile de chitosane.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2006042161 A2
- EP 1579876 A2
- WO 2006050951 A1
- WO 03035122 A1
- FR 2754268
- WO 9805366 A1

**In der Beschreibung aufgeführte Nicht-Patentliteratur**

- *Ph. Eur.,* 2004, vol. 4
- **B.T. HOFREITER ; B.H. ALEXANDER ; I.A. WOLFF.** *Anal. Chem.,* 1955, vol. 27, 1930ff